# EUROPEAN PATENT SPECIFICATION

(11) **EP 4 061 204 B1**
(45) Date of publication and mention of the grant of the patent: **28.10.2026**
(21) Application number: 20823733.9
(22) Date of filing: 18.11.2020
(51) Int. Cl.: A61B 5/00, A61B 5/03, A61B 5/026

(54) **DEVICE AND METHOD FOR NON-INVASIVE PREDICTION OF INTRACRANIAL PRESSURE BASED ON OPTICAL MEANS**
VORRICHTUNG UND VERFAHREN ZUR NICHT-INVASIVEN VORHERSAGE DES INTRAKRANIELLEN DRUCKS AUF DER BASIS OPTISCHER MITTEL
DISPOSITIF ET PROCEDE DE PREDICTION NON-INVASIVE DE LA PRESSION INTRACRANIERE A PARTIR DE MOYENS OPTIQUES

(30) Priority: 18.11.2019 GB 201916782
(43) Date of publication of application: 28.09.2022
(73) Proprietor: City, University of London, London EC1V 0HB (GB)
(72) Inventor: KYRIACOU, Panayiotis, London, Northampton Square; London EC1V 0HB (GB); ABAY, Tomas, London, Northampton Square; London EC1V 0HB (GB); PHILLIPS, Justin, London, Northampton Square; London EC1V 0HB (GB); UFF, Christopher, London E1 1BB (GB)
(74) Representative: Milhench, Mark Lorne
(86) International application number: PCT/EP2020/082598
(87) International publication number: WO 2021/099426

(56) References cited:
- US-A1- 2009 234 245
- US-A1- 2011 201 962
- US-A1- 2016 058 395
- US-A1- 2016 192 849
- US-A1- 2018 103 861
- DAVIDE TAMBORINI ET AL: "Development and characterization of a multidistance and multiwavelength diffuse correlation spectroscopy system", NEUROPHOTONICS, vol. 5, no. 01, 21 September 2017 (2017-09-21), 1000 20th St. Bellingham WA 98225-6705 USA, pages 1, XP055768743, ISSN: 2329-423X, DOI: 10.1117/1.NPh.5.1.011015
- FARZAM PARISA ET AL: "Fast diffuse correlation spectroscopy (DCS) for non-invasive measurement of intracranial pressure (ICP) (Conference Presentation)", PROGRESS IN BIOMEDICAL OPTICS AND IMAGING, SPIE - INTERNATIONAL SOCIETY FOR OPTICAL ENGINEERING, BELLINGHAM, WA, US, vol. 10050, 8 February 2017 (2017-02-08), pages 100500U - 100500U, XP060084326, ISSN: 1605-7422, ISBN: 978-1-5106-0027-0, DOI: 10.1117/12.2252824
- ALEXANDER RUESCH ET AL: "Intracranial Pressure Changes Derived from Near Infrared Spectroscopy Measurements in Non-Human Primates", BIOPHOTONICS CONGRESS: BIOMEDICAL OPTICS CONGRESS 2018 (MICROSCOPY/TRANSLATIONAL/BRAIN/OTS), 1 January 2018 (2018-01-01), Washington, D.C., pages OW4C.3, XP055768471, ISBN: 978-1-943580-41-5, DOI: 10.1364/OTS.2018.OW4C.3
- ALEXANDER RUESCH ET AL: "Diffuse correlation spectroscopy for intracranial pressure estimation through cardiac pulse waveform analysis", DIFFUSE OPTICAL SPECTROSCOPY AND IMAGING VII, vol. 11074, 11 July 2019 (2019-07-11), pages 20, XP055768614, ISBN: 978-1-5106-2842-7, DOI: 10.1117/12.2527187

## Description

### Field

This disclosure relates to monitoring systems, particularly but not exclusively to systems for monitoring intracranial pressure and other physiological parameters. In one illustrative implementation, the system is operable to continuously and non-invasively monitor intracranial pressure.

In one illustrative application, the system of a preferred embodiment is particularly useful in the context of patients presenting with head injuries, and in the following detailed description particular emphasis will be placed on this particular application of the teachings disclosed herein. It is important to remember, however, that this application is merely illustrative, and that the system embodying the teachings disclosed herein could also facilitate research and clinical monitoring in non-head injury medicine, such as liver failure, migraine, diabetes, anaesthesia, intensive care, renal medicine etc.

### Background

Head injuries are a significant cause of injury and death, with approximately 50,000 cases of severe traumatic brain injury per year in the UK, the majority leading to death or severe disability. Cerebral damage sustained at the time of impact is referred to as primary injury and is irreversible and best treated by prevention (seatbelts, cycle helmets etc). Secondary brain injury occurs after the initial injury and is defined as damage arising from the body's physiological response to the primary injury. This may be as a result of bleeding or swelling of brain tissue.

As the skull is a closed cavity containing water and other largely incompressible material, even minor swelling can cause significant increase in intracranial pressure (ICP).

Initially, cerebrospinal fluid and venous blood are displaced (as described in the Munro-Kellie doctrine) but once these reservoirs are exhausted, small increases in pressure are transmitted directly to the brain tissue, compromising the arterial blood supply and reducing oxygen and glucose delivery to the brain tissue. This in turn results in further brain swelling which further compromises blood supply. Severe hypoxic brain injury can result, leading to irreversible brain damage.

Various strategies exist to arrest or reverse this process and in that connection, monitoring ICP is a vital tool in the management of severe head injuries. The "gold standard" technique for ICP monitoring is a catheter inserted into the frontal horn lateral ventricle via a right frontal burr hole, connected to a pressure transducer via a fluid-filled catheter. This arrangement has the advantage of allowing therapeutic drainage of cerebrospinal fluid (CSF) and administration of drugs. It is the case, however, that insertion may be difficult if the ventricles are small, and even if performed in a sterile environment infection occurs in approximately 11% of patients. Ventricular catheters measure global ICP and have the additional advantages of allowing periodic external calibration. Most clinicians now use electrical or fibre optic pressure transducers that are inserted into the brain tissue in the right frontal lobe via a twist drill hole (which is smaller than a burr hole). Although these are easier to insert and carry a lower infection risk, they are prone to drift and despite being less invasive than a ventricular catheter, they still carry a small risk of causing significant intracranial bleeding.

Whilst there has been much research in recent years to find a method for measuring intracranial pressure noninvasively (nICP) (including measurement of pressure in the retinal veins, measurement of eardrum displacement, transcranial Doppler ultrasonography and imaging-based solutions), it is the case that none of these methods have found their way into clinical use as they all require considerable user intervention and are non-continuous.

A recent review in Nature Reviews: Neuroscience reported that ICP monitoring is still a standard of care for traumatic brain injury patients and that there has been much recent interest in the potential of new non-invasive ICP monitoring techniques. Methods using tympanic membrane displacement and ultrasound "time of flight" techniques have been described, and whilst both are a poor surrogate for invasive ICP measurements, serial intra-patient measurements may be useful to determine temporal changes in ICP.

More recently, transcranial Doppler ultrasonography has been used to provide an indirect estimation of cerebral perfusion pressure ((CPP) i.e. the difference between mean arterial blood pressure and ICP) to an accuracy of ±10-15 mmHg, however these systems are expensive and as they require user intervention, they do not provide continuous monitoring and can only be used in a high dependency hospital environment.

It is estimated that in the UK, approximately 1 million patients attend hospital with a head injury per year, or which 5% are classed as severe. Of those with severe TBI, over 85% remain disabled after one year and 15% have not returned to work after five years. Traumatic injury kills more people below the age of 45, than any other cause, accounting for 18,000 deaths per year in the UK. Road traffic accidents are the most common cause of head injuries and are especially common in teenagers and young adults.

The care received by the patient (in the 'golden hour') immediately after injury may profoundly affect the outcome. This has resulted in the inception of pre-hospital medicine where care is directed towards ABC (securing the airway, oxygenating the patient and maintaining circulation), however little thought is given to head injury until the patient arrives in hospital. Even after arrival in a specialist hospital it may be up to an hour before definitive imaging is performed and surgery or ICP directed therapy is instituted. This paradigm is changing and pre-hospital SOPs (Standard Operating Procedures) now administer drugs to reduce ICP if there is evidence of a head injury, but this is a blind treatment. Pre hospital or immediate in-hospital ICP monitoring would allow ICP directed therapy from the outset.

There is significant evidence to suggest that therapy directed to maintain brain tissue oxygenation as well as ICP/CPP is associated with reduced mortality after severe TBI. Multimodality intracranial monitoring is now widely used during neurointensive care to provide early warning of impending brain ischemia and guide targeted therapy to improve cerebral perfusion and oxygenation.

Despite its limitations, ICP monitoring remains central to the monitoring and management of severe TBI. Conventional approaches to management have concentrated on a reduction in ICP to prevent secondary cerebral ischemia. Treatment is usually initiated if ICP increases >20 mm Hg, although it is likely that the duration of intracranial hypertension and its response to treatment are also important prognostic indicators.

Advances in MRI functional imaging have brought a revolution in our understanding of the brain. In particular, the mechanism of spinal cord and brain injury is much better understood, however this knowledge has not yet led to significant improvements in injury management, partly due to lack of clinical monitoring data at the bedside. Intensive care and emergency medicine are also in a state of flux with increased emphasis on goal directed therapy and realisation of the importance of early intervention to recovery and survival rates.

Aspects of the arrangements disclosed herein have been devised with the foregoing problems in mind.

United States Patent Publication no. 2016192849 discloses a non-invasive pressure monitoring system which includes a first sensor placed proximate to a perfusion field of an artery receiving blood which emanates from the cranial cavity. The system is configured to measure pulsations of the artery receiving blood which emanates from the cranial cavity artery and generate first output signals. A second sensor is placed proximate to a perfusion field of an artery which does not receive blood emanating from the cranial cavity configured to measure pulsations of the artery which does not receive blood emanating from the cranial cavity and generate second output signals. A third sensor is configured to measure one or more physiological parameters of the human body and generate third output signals. A processing system responsive to signals from the first, second, and third output signals is configured to determine intracranial pressure.

United States Patent Publication no. 2018103861 discloses a system and method for non-invasively monitoring intracranial pressure. In some aspects, the method comprises acquiring diffuse correlation spectroscopy data at a temporal resolution greater than a pulsatile frequency of cerebral blood flow of a subject using one or more optical sensors placed about a subject, and determining a pulsatile cerebral blood flow using the acquired data. The method also includes correlating the determined pulsatile cerebral blood flow with physiological data acquired from the subject, and estimating an intracranial pressure based on the correlation. The method further includes generating a report indicative of the estimated intracranial pressure.

United States Patent Publication no. 2009234245 discloses methods, systems, and related computer program products for non-invasive detection of intracranial pressure (ICP) variations in an intracranial compartment of a patient. Optical radiation is propagated transcranially into the intracranial compartment, and optical radiation that has migrated through at least a portion of the intracranial compartment and back out of the cranium is detected. At least one signal representative of the detected optical radiation is processed to extract therefrom at least one component signal that varies in time according to at least one of an intrinsic physiological oscillation and an externally driven oscillation in the patient. Examples of suitable intrinsic physiological oscillations include intrinsic respiratory and cardiac oscillations. Examples of suitable externally driven oscillations include ventilated respiratory oscillations and externally mechanically induced oscillations. The extracted component signal is then processed to generate an output signal representative of the ICP variations in the intracranial compartment.

### Summary

In accordance with a presently preferred arrangement, there is provided an in-vivo intracranial pressure monitoring system, the monitoring system being as defined in Claim 1. Various preferred features of arrangements disclosed herein are set out in the accompanying dependent claims.

### Brief Description of the Drawings

Various aspects of the teachings disclosed herein, and arrangements embodying those teachings, will hereafter be described by way of illustrative example with reference to the accompanying drawings, in which:
Fig. 1 is a schematic representation of a non-invasive probe for a monitoring system embodying the teachings of the present invention;
Fig. 2(a) is an illustrative representation of absorbance calculated from the signals detected at the first detector D1;
Fig. 2(b) is an illustrative representation of differential absorbance calculated by subtracting the D1 detector signal from the D2 detector signal;

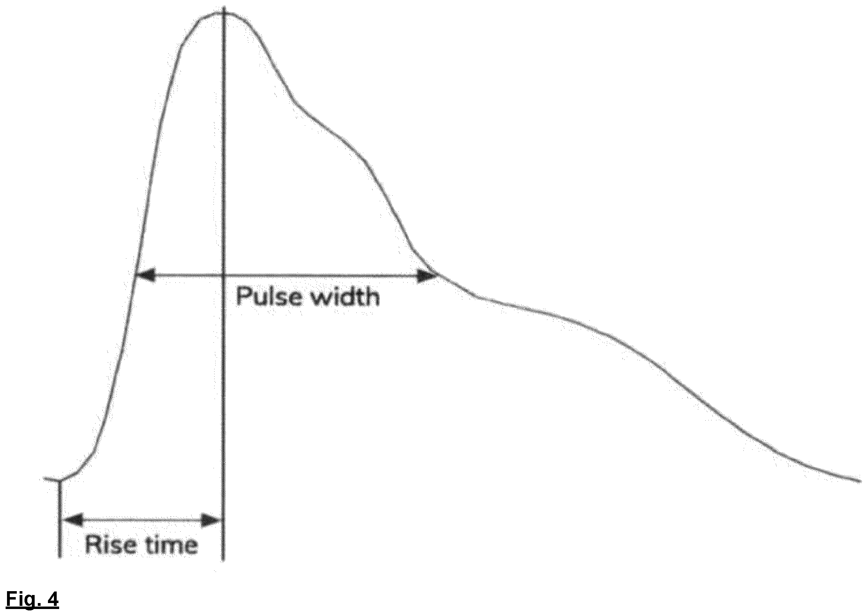

Figs. 3 and 4 are diagrammatic representations of illustrative features of an absorbance signal;
Fig. 5 is a diagrammatic representation of diastolic peak area;
Fig. 6(a) is a diagrammatic representation of other features of the absorbance signal;
Fig. 6(b) is a diagrammatic representation of a first derivative of the signal shown in Fig. 6(a);
Fig. 7 is a diagrammatic representation of a multi-variable regression model;
Fig. 8 is a diagrammatic representation of a process for generating the prediction model of Fig. 7;
Fig. 9 is a diagrammatic representation of a process for generating real-time intracranial pressure predictions;
Fig. 10 is a schematic representation of an illustrative controller for use with the system disclosed, and
Fig. 11 is a schematic representation of illustrative software modules for implementing some or all of the functionality disclosed herein.

### Detailed Description

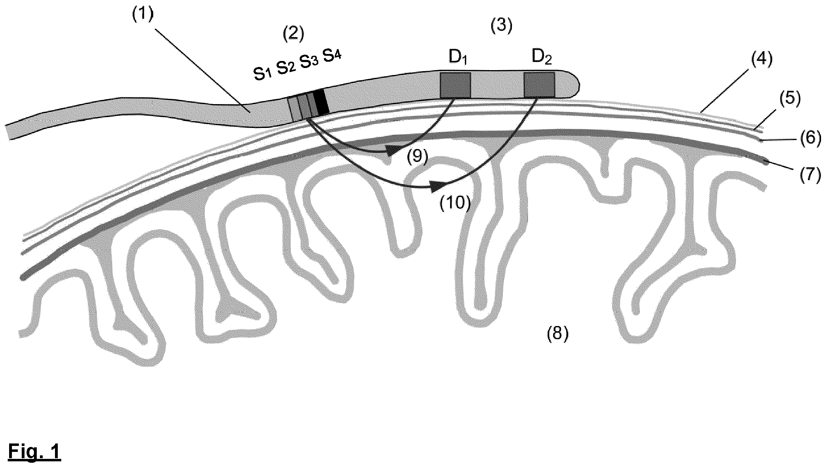

Referring now to Fig. 1, there is depicted a schematic representation of a non-invasive probe (1) for use with a monitoring system embodying the teachings of the present disclosure.

The probe (1) contains multiple near-infrared/infrared sources (2), each capable of emitting light of a different wavelength or band of wavelengths. The sources can be switched on and off as needed. A pair of detectors (3) is also contained within the probe. The detectors are spaced from one another within the probe - for example by a few millimetres to one or more centimetres.

In a preferred implementation, the sensor contains an array of four optical sources that are capable of emitted light of different wavelengths. In this arrangement the sources comprise light emitting diodes emitting light of four different wavelengths in the near infrared/infrared. The two detectors (3) comprise photodetectors (for example, photodiodes) located at different distances from the sources. In one implementation the first detector D1 is 1 cm from the sources and the second detector D2 is 3.5 cm from the sources. In this particular example, the sources are operable to emit light of 810 nm, known as the principal wavelength, and three other secondary wavelengths 770 nm, 855 nm and 880 nm. Light having a wavelength in the range between 805-810 nm is at the isobestic point, where oxygenated and deoxygenated haemoglobin have the same optical absorption properties. As such, this principal wavelength provides signals that are independent from the oxygenation of blood. In other implementations light of 780, 805, 850 and 870 nm may be emitted in place of or in addition to any of the aforementioned wavelengths.

The probe (1) is secured to the forehead epidermis (4) of a patient, and the infrared sources are operable to illuminate the dermis (5), skull (6), dura mater (7), and the frontal lobe of the brain (8) (part of cerebral cortex). Reference numerals (9) and (10) indicate, respectively, the mean optical path between a given source and a proximal detector (D1) of said pair of detectors (3); and the mean optical path between a given source and a distal detector (D2) of said pair of detectors. In a preferred arrangement multiplexing and de-multiplexing the sources and detectors results in eight acquired signals (2 detectors x 4 wavelengths) which are reduced to four signals representing the absorbance of brain tissue at each of the aforementioned wavelengths.

Light from one of the sources (e.g. S2) penetrates the tissue and is detected by both detectors. The proximal detector (D1) detects light from the superficial (extra-cerebral) layers (4-7), while the distal detector (D2) detects light from the superficial and deeper brain (cerebral) layers (4-8).

The probe is coupled to a controller (not shown) that is configured to send signals to the probe (1) to activate and deactivate the sources (2), and to receive signals from the detectors (3). The controller is operable to process signals received from the detectors, in particular to calculate the degree to which light input into the skull of the patient is absorbed by the superficial and deeper brain layers.

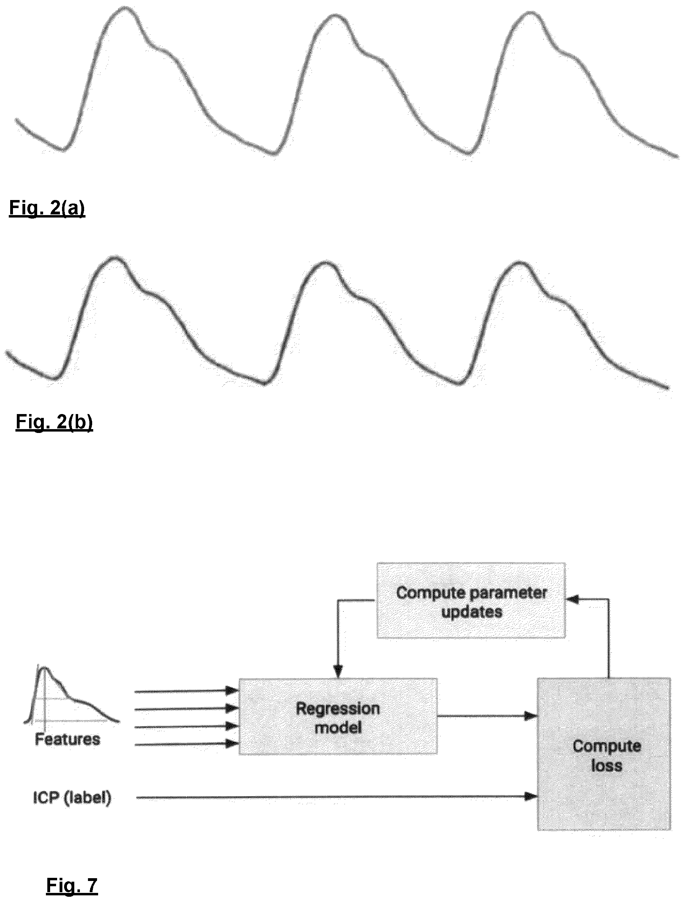

Absorbance of light is calculated from the light intensity detected by D1 and D2 using the Beer-Lambert Law (A = In(lo / I) where lo and I are incident and detected intensity respectively). Subtracting the absorbance signal from D1 from the absorbance from D2 gives absorbance signal for deep brain tissue only (since this represents the difference in optical path through the tissue). Thus the effects of extra-cerebral tissue from the optical path is reduced (ideally, substantially eliminated). Alternatively, signal processing techniques such as independent component analysis or blind signal separation can be used to subtract absorbance D1 from the absorbance of D2, using the absorbance at D1 as reference signal. Fig. 2(a) is an illustrative representation of the absorbance calculated from the signals detected at the first detector D1, and Fig. 2(b) is an illustrative representation of the differential absorbance calculated by subtracting the D1 detector signal from the D2 detector signal. The depth of penetration of the light may be controlled by selecting appropriate wavelengths of light (by switching on a particular source) because longer wavelengths of near-infrared light penetrate tissue more deeply than short wavelengths. Alternatively, a larger emitter-detector separation distance >3.5 cm can be used to increase penetration. As will be appreciated, light detected by the detectors will be modulated by pulsation of the cerebral arteries. Changes in the pressure surrounding the cerebral arteries will thus affect the morphology and other characteristics of the recorded optical pulse, and the controller is configured to analyse certain quantifiable features of the acquired signal and thus generate a measure of non-invasive ICP (nICP).

In an envisaged arrangement, the controller is coupled to a display for displaying a generated measure of nICP to clinicians, and may be configured to provide warnings (for example, a visual and or sonic warning) in the event that nICP changes, for example exceeds a predetermined threshold.

In an envisaged implementation, the controller may comprise a portable processor, for example a so-called laptop computer. Alternatively, the controller may be configured as a dedicated portable device. Such an implementation would provide a particularly useful tool for paramedics or doctors working on a patient outside of a hospital. In another envisaged implementation, the controller may be incorporated into other types of monitoring apparatus for use inside of a hospital. As will be appreciated by persons of ordinary skill in the art, the functionality provided by the controller may be implemented - at least partly - by one or more software modules.

Inside the cranial cavity the brain is surrounded by cerebrospinal fluid. The cerebral vasculature includes arterioles, venules and capillaries. Since the cranial cavity is rigid, the total volume is constant. Arterioles are thick walled vessels containing layers of smooth muscle, containing blood at variable pressure. Changes in arteriolar volume due to the pulse create inverse changes in the venule and capillary volumes (since brain tissue and cerebrospinal fluid are incompressible). Arterial pulsation is detectable using light in the -900 nm region. The pulsation of venules and capillaries show inverse pulsation, detectable using 750 nm light.

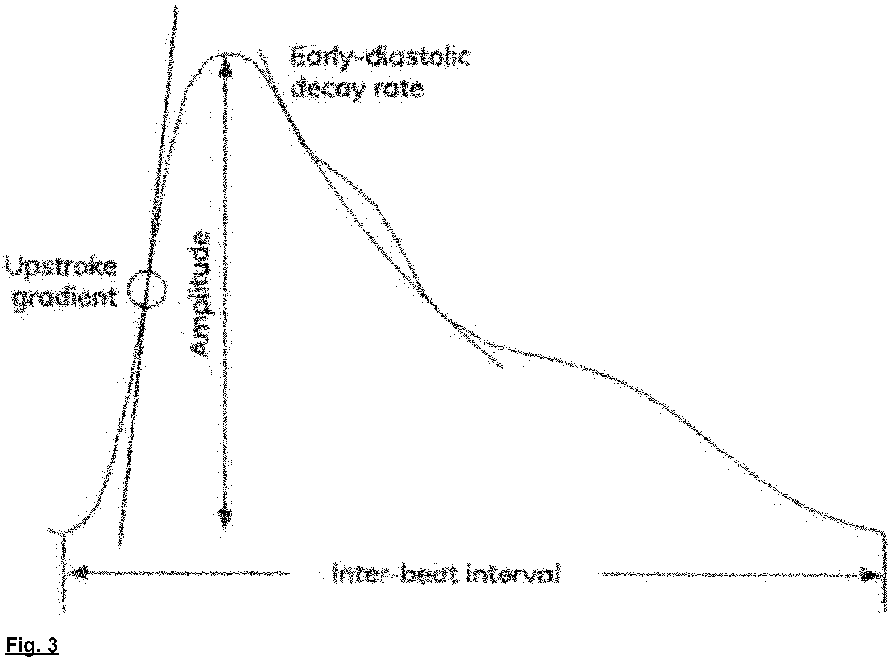

Several features are extracted from each pulse waveform and averaged over a rolling-window of, in this particular implementation, approximately 15 seconds. These features are shown in Figs. 2 and 3 of the accompanying drawings, and there follows below a brief explanation of their relation to intracranial pressure values. Note that all the features discussed below are applicable to the 805-810 nm waveform (i.e. the signal obtained at the principal-isobestic wavelength) unless otherwise stated.

### Pulse amplitude

The pulse amplitude (i.e. the height of the wave from foot to peak), normalised dividing by total light intensity is positively correlated with ICP. This is because the intracranial pressure facilitates emptying of the arterioles during diastole (effectively lowering the 'foot' of the wave). We have determined, except at very high ICP values, that the amplitude pulse amplitude varies approximately linearly with ICP.

### Upstroke gradient

Upstroke (or upslope) gradient is positively correlated with ICP, since the arterioles are emptier at the beginning of systole when ICP is elevated compared with normal values.

### Rise time

The rise time is normalised dividing by the inter-beat interval. We have determined that the rise time (time taken to reach peak amplitude) is positively correlated with ICP for the reason stated for the upstroke gradient above, i.e. end-diastolic emptying of arterioles requires longer filling time during systole. Note that increased ICP causes the peak of the waveform to arrive slightly earlier (since expansion of the vessel ceases when internal and external pressures become equal), which may decrease correlation of rise time with ICP to some extent.

### Early diastolic decay rate (EDDR)

The decay rate can be found by fitting an exponential function to the diastolic runoff (downstroke) of the waveform and is normalised dividing by the peak amplitude. We have determined that the decay rate value is positively correlated with ICP because elevated ICP facilitates faster emptying of the arterioles.

### Pulse width (negative correlation with ICP)

The pulse width, measured at the mid-point between the foot and peak of the wave on the y-axis, is negatively correlated with ICP for the reason stated for EDDR, i.e. because elevated ICP facilitates faster emptying of the arterioles.

### Late diastolic area under curve

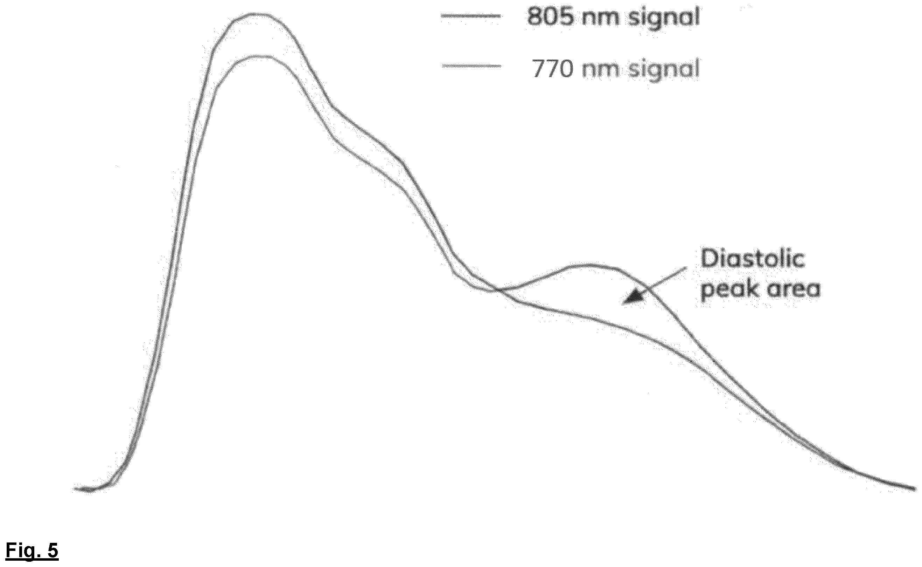

A secondary peak appearing in the later part of the waveform relates to filling of the venules from capillary blood as well as back-filling from larger veins when supine (cerebral veins do not contain valves to prevent this unlike most systemic veins). The process is impeded by elevated ICP, since the ICP exerts pressure on the exterior wall of the vessels preventing them from filling. The area under the later part of the curve, normalised by dividing by the total area under the pulse is indicative of the volume of the pulse. Comparison of the waveforms obtained at the principal wavelength and a shorter wavelength (see Figure 5) provides an estimation of the area of the secondary peak since venous blood contains significant deoxyhaemoglobin, which absorbs shorter wavelength near infrared light more strongly than longer wavelengths.

Note that filling of the arterioles during systole causes emptying of the venules (as well as capillaries), effectively causing pulsation of the venules. This has been observed in the brain as well as in non-cerebral tissue.

### Total backscattered intensity

The total backscattered intensity is indicative of blood volume. Elevated ICP effectively 'squeezes' blood from the brain, leading to less absorption of light passing through the brain tissue and higher detected intensity. The intensity is therefore positively correlated with ICP. This quantity should be averaged over the length of the sampling window (for example, c. 15 s).

### Decay time

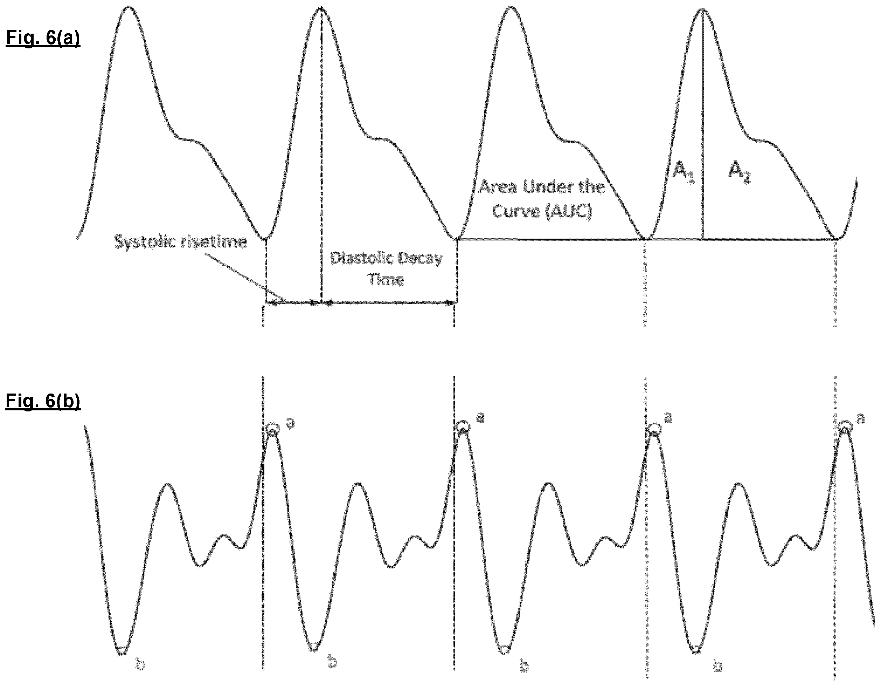

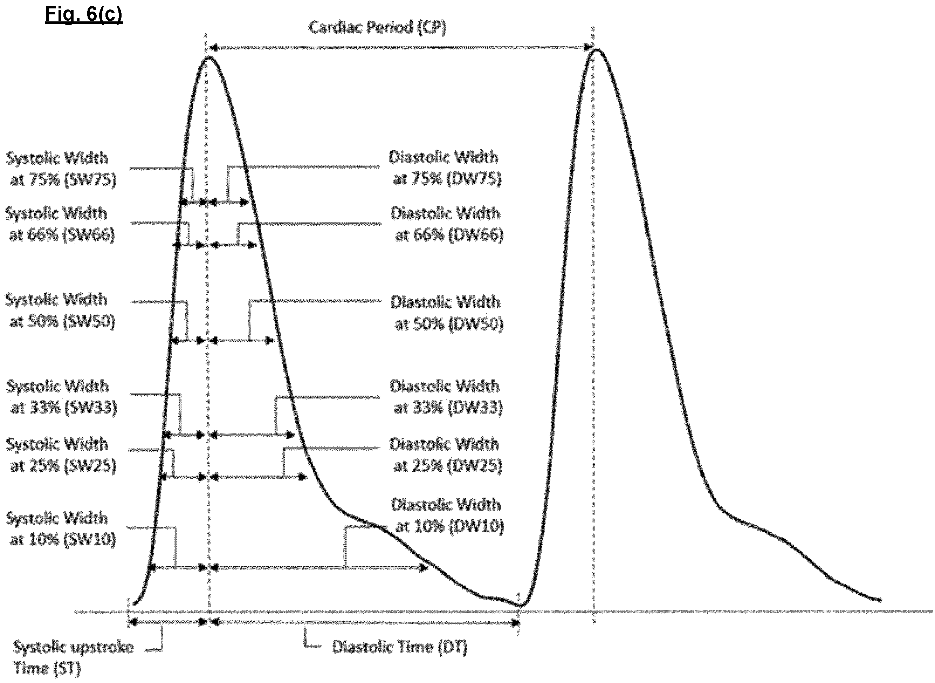

As shown in Fig. 6(a), the time from the pulse's peak to the pulse's valley. Also defined as the time (duration) of the diastolic period of the pulse.

### Area Under the Curve (AUC)

The area under the entire pulse's profile or waveform (see Fig. 6(a)).

### AUC Ratio

The AUC ratio is defined as the ratio between the area under the curve of the systolic period of the pulse (A1) and the area under the curve of the diastolic period (A2). (AUC Ratio = A2/A1), and is used as an indicator of vascular resistance, which varies with intracranial pressure.

### Second Derivative Pulse Ratio

With reference to Fig. 6(b), the second derivative pulse ratio is defined as the ratio of the point b and point a of the first derivative of the pulse signal and is representative of an indicator of arterial stiffness, which tends to vary with intracranial pressure.

Other features of interest are set out in the table below.

**Table 1: Illustrative features extractable from pulse waveform**

| **Feature** | **Calculation** |
|---|---|
| **PPG raw** | |
| Systolic amplitude - X | systolic peak |
| Diastolic amplitude - Y | diastolic peak |
| Dicrotic notch - Z | dicrotic peak |
| pulse widths @: | |
| at 10% | DW10; DW10+SW10; DW10/SW10 |
| at 25% | DW25; DW25+SW25; DW25/SW25 |
| at 33% | DW33; DW33+SW33; DW33/SW33 |
| at 50% | DW50; DW50+SW50; DW50/SW50 |
| at 66% | DW66; DW66+SW66; DW66/SW66 |
| at 75% | DW75; DW75+SW75; DW75/SW75 |
| systolic area A1 | systolic area |
| diastolic area A2 | diastolic area |
| Pulse area or inflection point ratio (A2/A1) | diastolic area/ systolic area |
| peak to peak interval | time difference between 2 consecutive peaks |
| Pulse interval time (PI) | pulse time from foot to end of waveform |
| ratio PI/X | PI /X |
| Heart rate | 60/PI |
| Augmentation Index | Y/X |
| Large Artery Stiffness Index | (h/ΔT) |
| Crest time or systolic upstroke time (ST) | time from foot to the peak |
| Diastolic time (DT) | time from peak to end of cycle |
| ratio of ST over PI time | ST/PI |
| ratio of DT over PI time | DT/PI |
| Dicrotic notch time | |
| ΔT | time diff between x and y |
| main wave rising slope (max_s) | values of the PPG at first deriv peak index |
| Relative height of max slope point | Height_max_s/X |
| time between the max slope index and peak (T_max_slope) | time between max slope index and peak index |
| ratio of T_max_slope over PI | T_max_slope/ PI |
| diastolic peak falling slope | y/(PI- dicrotic peak time) |
| PPG Intesity Ratio (PIR) | Ratio of PPG peak intensity to PPG bottom intensity (X/ppg_foot) |
| PPG_K value | k = (PPG_m - PPG_foot)/(PPG_peak - PPG_foot) where PPG_m = (1/PI) ∫ PPG(t)dt |

| **First derivative** | |
|---|---|
| ΔT | time diff between the two positive to negative zero crossing. Or between PPG peak and local max |
| peak amplitude (a1) | peak height |
| first peak time (t_a1) | time from foot to peak |
| valley time (t_b1) | time from peak to local minima (first valley) |
| ratio peak time to PI | t_a1/PI |
| ratio t_b1 to PI | t_b1/PI |
| second peak time (t_e1) | time from foot to second local maxima (occurs after the first valley) |

| **Second derivative** | |
|---|---|
| a value- peak | peak intensity -height |
| b- foot height | foot intensity |
| ratio A_b/ A_a | A_b/ A_a |
| ratio AP_b/AP_a | amplitude of AP_b over AP_a |
| ratio A_c/ A_a | |
| ratio A_d/ A_a | |
| ratio A_e/ A_a | amplitude of A_e over A_a |
| AP_e/AP_a | amplitude of AP_e over AP_a |
| peak time t_a2 | time from foot to peak |
| time interval between a and b (t_b2) | time from peak to local minima |
| time interval between b and e (t_c2) | time from local minima to peak point e |
| total intensity | height between peak and local minima |
| ratio peak time to PPG PI | t_a2 / PI |
| ratio t_b2 to PI | t_b2/ PI |
| ratio t_c2 to PI | t_c2/PI |

The intracranial pressure can be calculated, for example by the aforementioned controller, using a simple algorithm based on a small number of features, selected from those that show the best correlation with ICP. A simple linear or nth-order function, calibrated from clinical trial data, may be used to convert features into ICP values.

In another envisaged arrangement depicted schematically in Fig. 7, an algorithm based on, for instance, multiple variable regression may be employed. Features from the acquired waveforms (such as one or more of the aforementioned features) are input into a model (prediction function) trained on data obtained in a clinical trial and labelled with target ICP values obtained using a gold standard method (such as an invasive ICP monitor). The weights of the inputs of the model are adjusted during training to produce the lowest error (cost function) between the predicted and target ICP values.

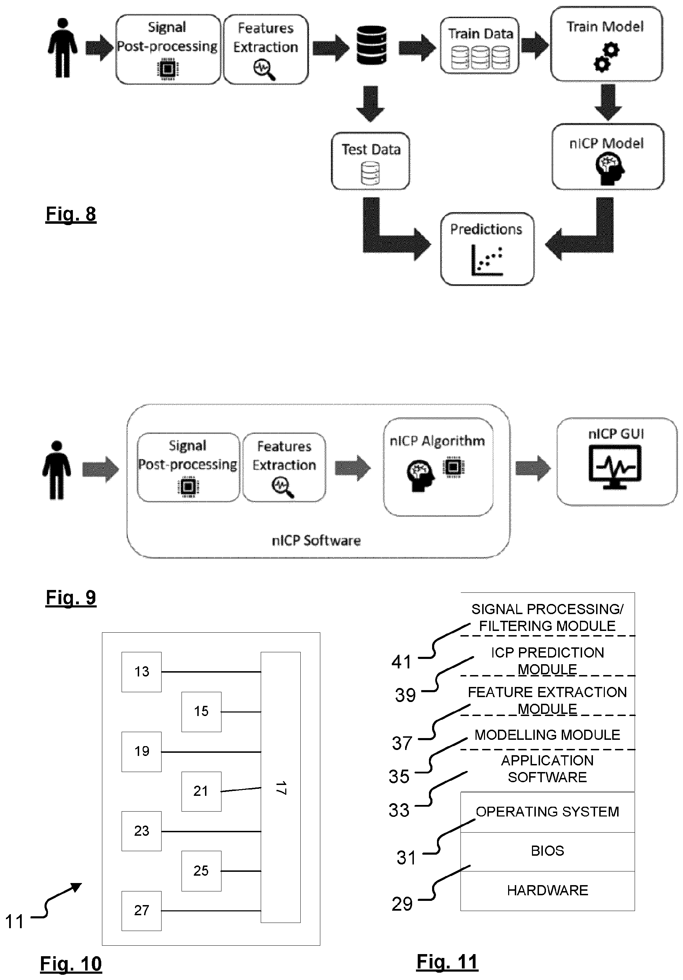

Fig. 8 is a schematic representation of an illustrative process for establishing the aforementioned model.

Once the multi-wavelength optical signals are acquired from the patient's head, they undergo a post-processing stage. At this stage, the optical signals are filtered to remove noise and baseline drifts by means of digital signal processing filtering techniques. After filtering out noise, the pulsatile signals are then normalised by their DC baseline values (also filtered from the raw optical signals by low-pass filters). This normalisation process enables the ability to take into account of the proportion of total light absorbed, which may differ within patients, wavelengths or within measurements.

After signal filtering and normalisation, one or more of the features described above are extracted from the signal. The feature extraction can be performed in a rolling window with no prefixed length. As changes in the intracranial pressure (absolute) values are monitored for rather longer period, this rolling window's length can be from few seconds up to 2 minutes. Once the features are extracted within the rolling window, considering that a rolling window will contain more than one pulse, the features extracted for each pulse can be averaged across the number of pulses (i.e. one feature value for each rolling window).

After feature extraction and averaging within each rolling window, the extracted feature(s) are passed to a post-processing stage. In this stage, the features extracted are standardised to transform them into a gaussian (normal) distribution and to take into account of magnitude/scale differences between features. After standardisation, the *n* number of features extracted go a transformation process to maximise the variance captured.

The resultant data are then split into training and test data (at a discretionary ratio), following common Machine Learning techniques. The training data (usually 70-80% of total data) are used to train the prediction model, whereas the remaining data is held to later validate the performance of the model.

The model is then trained/built using supervised Machine Learning, where the target (known) intracranial pressure is used to construct a model that will predict the response variable (ICP) based on the input predictors (features). The prediction model will try to fit the response variable and predictors whilst minimising the error of prediction. In one illustrative implementation, the model is based on Support Vector Machines algorithms. However, another model among the many available, for instance Neural Network, could also be utilised.

After the model has been trained, it is then validated using the 'unseen' test data to verify the accuracy of prediction and, if required, optimise or change the model. Other validation techniques such as k-fold crossvalidation or Leave One Out Crossvalidation (LOOCV) can be used to validate the model.

Once the prediction model has been finalised and validated, it can be implemented into a 'live' process implementing an algorithm/software that predicts intracranial pressure measurements in real-time, as shown in Fig. 9.

Different optical signals can be used to predict the intracranial pressure. The optical probe enables the collection of light at multiple (in this instance, four) different wavelengths, hence different options are available for this purpose. One illustrative option is to use a single wavelength at the isobestic point to extract the relevant features for intracranial pressure prediction. The advantage of using the isobestic wavelength is its independence from blood oxygenation. Another option is to obtain a pulsatile signal from the pulsatile component of the oxygenated haemoglobin (HbO2). By using two of the wavelengths, the oxygenated and deoxygenated haemoglobin signals can be spectroscopically separated. From this, the HbO2 can be used to extract the relevant intracranial pressure features.

Fig. 10 is a schematic representation of an illustrative controller 11 for use with aspects of the system disclosed. As aforementioned, the controller may advantageously comprise a laptop computer or other portable computing device.

The controller 11 comprises a power supply unit 13 that is configured to draw power from a mains power supply and regulate the supply of power to the remaining components of the controller. The controller 11 includes a processor 15 that is coupled to a system bus 17 by means of which signals can be sent between the processor and the other components of the controller. The controller 11 further comprises read only memory (ROM) and/or random access memory (RAM) 19 that provides a processing environment in which the processor 15 can execute computer programs. The controller also includes a data store 21 for the storage of computer programs for execution by the processor.

In this illustrative embodiment, the system bus 17 is coupled to a communications interface 23 (for example, an Ethernet and/or wireless interface), a peripheral interface 25 and a video controller 27. The peripheral interface 25 is configured to enable user interface devices, such as a keyboard and/or pointing device (such as a mouse or trackball), and ancillary equipment such as one or more printers to be connected to the hub for use therewith. The peripheral interface could include RS232 connectors, USB connectors, PS2 connectors or any other type of connector. The video controller 27 provides an interface that enables a display, not shown, to be coupled to the hub, and functions in response to signals from the processor to generate images for display on the display.

In the preferred arrangement, much of the functionality herein described is implemented in software, but it will be appreciated by persons skilled in the art that some or all of this functionality could alternatively be implemented in hardware (for example by means of one or more application specific integrated circuits (ASICs)) or by means of a combination of hardware and software. As such, the scope of the present invention should not be interpreted as being limited only to being implemented in software.

Referring now to Fig. 11, in the preferred arrangement the processor 15 and memory 19 cooperate to establish a BIOS (Basic Input/Output System) 29 that functions as an interface between the functional hardware components 31 of the controller and the software executed by the controller. The processor then loads from memory 19 an operating system 31 which provides an environment in which application software 33 (implementing some or all of the abovedescribed functionality) can run. In accordance with the preferred embodiment of the present invention, part of this functionality is provided by a modelling module 35, a feature extraction module 37, an intracranial pressure prediction module 39, and a signal processing/filtering module 41, the functions of each of which have been outlined above.

The reported nICP provides invaluable screening at the triage stage, indicating intracranial hypertension requiring imaging or intervention (such as CSF drainage). It also provides effective guidance for head injury management, notably ICP-targeted treatment regimes. Ultimately this could lead to significant improvements in secondary injury-related mortality, length of hospital stay and reduced post-trauma disability. It also finds application in causes of non-trauma related intracranial hypertension including meningitis, hepatic encephalopathy, hydrocephalus and severe migraine.

As aforementioned, aside from trauma, management of other conditions associated with intracranial hypertension (such as hydrocephalus, severe migraine and meningitis) could benefit from nICP monitoring. In many cases, especially borderline cases or those in early stages, the risk of invasive ICP monitoring is not justifiable; nevertheless intracranial monitoring could provide invaluable clinical information. The aforementioned non-invasive ICP monitor would also be an invaluable research tool both for investigation of pathophysiology and for assessment of the effectiveness of treatments for intracranial hypertension.

In addition to the foregoing, the teachings disclosed herein may be employed to infer a number of additional physiological parameters, such as:
- Changes in cerebral blood volume (LICBV) - inferred from the change in total backscattered intensity.
- Heart rate - obtained from the inter-beat intervals averaged over the sampling window.
- Respiration rate - inferred by the frequency of periodic modulation of the pulse waveform.
- Haemoglobin concentrations estimated spectroscopically from the simultaneous multi-wavelength measurements.
- Oxy- and deoxygenated haemoglobin pulsations. As above, the raw multi-wavelength signals can be used to separate spectroscopically the 'pure arterial' and 'pure venous' pulsations.
- Low frequency oscillations can be extracted from the signals. These can be used to estimate reactivity indexes (against systemic arterial blood pressure) for assessing the state of cerebral autoregulation (correlated with ICP).

It will be appreciated that whilst various aspects and embodiments of the present disclosure have heretofore been described, the scope of the present invention is not limited to the particular arrangements set out herein and instead extends to encompass all arrangements, and modifications and alterations thereto, which fall within the scope of the invention as defined by the accompanying claims.

In addition, whilst certain arrangements have been described above in the context of software modules that are executable by a processor, it should be noted that the scope of the present invention is not limited to an implementation of the disclosure in software. Rather, the skilled person will immediately appreciate that the functionality described herein may equally be implemented in hardware (for example, by means of a plurality of application specific integrated circuits (ASICS)) or, indeed, by a mix of hardware and software.

## Claims

1. An in-vivo intracranial pressure monitoring system, the monitoring system comprising:
a probe (1) configured to be securable to a patient's forehead epidermis, the probe comprising a plurality of optical emitters (2) and a pair of optical detectors (3); the optical detectors (3) being configured to detect light emitted by the optical emitters (2) and to generate signals representative of said detected light; wherein a proximal detector (D1) and a distal detector (D2) of said pair of optical detectors are located in the probe so that the distal detector (D2) is further from the plurality of optical emitters than the proximal detector (D1), the arrangement being such that, in use, said proximal detector (D1) detects light from the optical emitters (2) that has passed through extra-cerebral layers of the patient's head and said distal detector (D2) detects light from the optical emitters (2) that has passed through extra-cerebral and cerebral layers of the patient's head; and
a controller (11) comprising memory (19) and a processor (15), the controller (11) being connectable to the probe (1) for energising the optical emitters (2) and receiving signals from the detectors (3), wherein the signals received from the detectors (3) are indicative of the degree to which light input into the subject's skull is absorbed by the extra-cerebral and cerebral layers, said controller (11) being configured to subtract the absorbance signal from the proximal detector (D1) from the absorbance signal from the distal detector (D2) to provide an absorbance signal for cerebral layers of the patient's brain, said cerebral layer absorbance signal being representative - when said probe is attached to a subject's head - of light from the emitters that has been modulated by pulsation of the subject's cerebral arteries, changes in pressure surrounding the cerebral arteries affecting the morphology and other characteristics of said cerebral layer absorbance signal;
the system further comprising a modelling module (35) configured to establish a model relating features of an optical signal to intracranial pressure, the optical signal being representative of a degree to which light input into a subject's skull is absorbed by the subject's brain (8), wherein said model comprises a prediction function trained on clinical trial data labelled with target ICP values obtained using a gold standard method, input weights of the model being adjusted during training to reduce error between predicted and target ICP values;
a feature extraction module (37) operable to extract one or more signal features from the cerebral layer absorbance signal derived from the optical signals output by said detectors (3), and
an intracranial pressure prediction module (39) operable to input said signal features into said model and output an indication of intracranial pressure in accordance with said model.

2. A system according to any preceding claim, wherein said emitters (2) are configured to capable of emitting infra-red/infra-red light.

3. A system according to Claim 2, wherein said optical emitters (2) are configured to emit light at a plurality of different wavelengths.

4. A system according to any preceding claim, wherein said optical emitters (2) are configured to emit light at a wavelength selected from one or more of 810 nm, 770 nm, 855 nm, 880 nm, 780 nm, 805 nm, 850 nm and 870 nm.

5. A system according to Claim 4, wherein the probe (1) comprises four emitters (2), a first said emitter (S1) being configured to emit light having a wavelength of approximately 810 nm, a second said emitter (S2) being configured to emit light having a wavelength of approximately 770 nm, a third said emitter (S3) being configured to emit light having a wavelength of approximately 855 nm, and a fourth said emitter (S4) being configured to emit light having a wavelength of approximately 880 nm.

6. A system according to any preceding claim, wherein said model is established by means of a multiple variable regression technique.

7. A system according to any preceding claim, wherein said feature extraction module (37) is configured to extract any one or more of the following features from said absorbance signal: pulse amplitude, upstroke gradient, rise time, early diastolic decay rate, pulse width, late diastolic area under the curve, and total backscattered light.

8. A system according to any preceding claim, further comprising a signal processing/filtering module (41) configured for processing and/or filtering the signals generated by said detectors (3).

## Patentansprüche

1. Ein System zur In-vivo-Überwachung des intrakraniellen Drucks, wobei das Überwachungssystem umfasst:
eine Sonde (1), die dazu eingerichtet ist, an der Stirnepidermis eines Patienten befestigt werden zu können, wobei die Sonde eine Mehrzahl optischer Emitter (2) und ein Paar optischer Detektoren (3) umfasst; wobei die optischen Detektoren (3) dazu eingerichtet sind, von den optischen Emittern (2) emittiertes Licht zu detektieren und Signale zu erzeugen, die das detektierte Licht repräsentieren; wobei ein proximaler Detektor (D1) und ein distaler Detektor (D2) des Paars optischer Detektoren so in der Sonde angeordnet sind, dass der distale Detektor (D2) von der Mehrzahl optischer Emitter weiter entfernt ist als der proximale Detektor (D1), wobei die Anordnung derart ist, dass im Gebrauch der proximale Detektor (D1) Licht von den optischen Emittern (2) detektiert, das extrazerebrale Schichten des Kopfes des Patienten durchlaufen hat, und der distale Detektor (D2) Licht von den optischen Emittern (2) detektiert, das extrazerebrale und zerebrale Schichten des Kopfes des Patienten durchlaufen hat; und
eine Steuereinheit (11), die einen Speicher (19) und einen Prozessor (15) umfasst, wobei die Steuereinheit (11) mit der Sonde (1) verbindbar ist, um die optischen Emitter (2) mit Energie zu versorgen und Signale von den Detektoren (3) zu empfangen, wobei die von den Detektoren (3) empfangenen Signale das Ausmaß anzeigen, in dem in den Schädel eines Subjekts eingebrachtes Licht von den extrazerebralen und zerebralen Schichten absorbiert wird, wobei die Steuereinheit (11) dazu eingerichtet ist, das Absorbanzsignal des proximalen Detektors (D1) von dem Absorbanzsignal des distalen Detektors (D2) zu subtrahieren, um ein Absorbanzsignal für zerebrale Schichten des Gehirns des Patienten bereitzustellen, wobei das Absorbanzsignal der zerebralen Schichten - wenn die Sonde am Kopf eines Subjekts angebracht ist - Licht der Emitter repräsentiert, das durch die Pulsation der zerebralen Arterien des Subjekts moduliert worden ist, wobei Änderungen des die zerebralen Arterien umgebenden Drucks die Morphologie und andere Eigenschaften des Absorbanzsignals der zerebralen Schichten beeinflussen;
wobei das System ferner umfasst: ein Modellierungsmodul (35), das dazu eingerichtet ist, ein Modell zu erstellen, das Merkmale eines optischen Signals mit dem intrakraniellen Druck in Beziehung setzt, wobei das optische Signal das Ausmaß repräsentiert, in dem in den Schädel eines Subjekts eingebrachtes Licht vom Gehirn (8) des Subjekts absorbiert wird, wobei das Modell eine Vorhersagefunktion umfasst, die anhand klinischer Studiendaten trainiert wurde, welche mit ICP-Zielwerten gekennzeichnet sind, die unter Verwendung eines Goldstandardverfahrens erhalten wurden, wobei Eingangsgewichte des Modells während des Trainings angepasst werden, um den Fehler zwischen vorhergesagten ICP-Werten und ICP-Zielwerten zu verringern;
ein Merkmalsextraktionsmodul (37), das dazu betreibbar ist, ein oder mehrere Signalmerkmale aus dem Absorbanzsignal der zerebralen Schichten zu extrahieren, das aus den von den Detektoren (3) ausgegebenen optischen Signalen abgeleitet wird, und
ein Modul (39) zur Vorhersage des intrakraniellen Drucks, das dazu betreibbar ist, die Signalmerkmale in das Modell einzugeben und gemäß dem Modell eine Angabe des intrakraniellen Drucks auszugeben.

2. System nach einem der vorhergehenden Ansprüche, wobei die Emitter (2) dazu eingerichtet sind, fähig zu sein, infrarotes/infrarotes Licht zu emittieren.

3. System nach Anspruch 2, wobei die optischen Emitter (2) dazu eingerichtet sind, Licht mit mehreren unterschiedlichen Wellenlängen zu emittieren.

4. System nach einem der vorhergehenden Ansprüche, wobei die optischen Emitter (2) dazu eingerichtet sind, Licht mit einer Wellenlänge zu emittieren, die aus einer oder mehreren der Wellenlängen 810 nm, 770 nm, 855 nm, 880 nm, 780 nm, 805 nm, 850 nm und 870 nm ausgewählt ist.

5. System nach Anspruch 4, wobei die Sonde (1) vier Emitter (2) umfasst, wobei ein erster der Emitter (S1) dazu eingerichtet ist, Licht mit einer Wellenlänge von ungefähr 810 nm zu emittieren, ein zweiter der Emitter (S2) dazu eingerichtet ist, Licht mit einer Wellenlänge von ungefähr 770 nm zu emittieren, ein dritter der Emitter (S3) dazu eingerichtet ist, Licht mit einer Wellenlänge von ungefähr 855 nm zu emittieren, und ein vierter der Emitter (S4) dazu eingerichtet ist, Licht mit einer Wellenlänge von ungefähr 880 nm zu emittieren.

6. System nach einem der vorhergehenden Ansprüche, wobei das Modell mittels eines Regressionsverfahrens mit mehreren Variablen erstellt wird.

7. System nach einem der vorhergehenden Ansprüche, wobei das Merkmalsextraktionsmodul (37) dazu eingerichtet ist, eines oder mehrere der folgenden Merkmale aus dem Absorbanzsignal zu extrahieren: Pulsamplitude, Steigung des Pulsanstiegs, Anstiegszeit, frühdiastolische Abklingrate, Pulsbreite, spätdiastolische Fläche unter der Kurve und gesamtes zurückgestreutes Licht.

8. System nach einem der vorhergehenden Ansprüche, ferner umfassend ein Signalverarbeitungs-/Filtermodul (41), das dazu eingerichtet ist, die von den Detektoren (3) erzeugten Signale zu verarbeiten und/oder zu filtern.

## Revendications

1. Système de surveillance in vivo de la pression intracrânienne, le système de surveillance comprenant :
une sonde (1) configurée pour pouvoir être fixée à l'épiderme du front d'un patient, la sonde comprenant une pluralité d'émetteurs optiques (2) et une paire de détecteurs optiques (3) ; les détecteurs optiques (3) étant configurés pour détecter la lumière émise par les émetteurs optiques (2) et générer des signaux représentatifs de ladite lumière détectée ; dans lequel un détecteur proximal (D1) et un détecteur distal (D2) de ladite paire de détecteurs optiques sont disposés dans la sonde de telle sorte que le détecteur distal (D2) est plus éloigné de la pluralité d'émetteurs optiques que le détecteur proximal (D1), l'agencement étant tel que, lors de l'utilisation, ledit détecteur proximal (D1) détecte la lumière provenant des émetteurs optiques (2) qui a traversé les couches extracérébrales de la tête du patient et ledit détecteur distal (D2) détecte la lumière provenant des émetteurs optiques (2) qui a traversé les couches extracérébrales et cérébrales de la tête du patient ; et
un contrôleur (11) comprenant une mémoire (19) et un processeur (15), le contrôleur (11) pouvant être connecté à la sonde (1) afin d'alimenter les émetteurs optiques (2) et de recevoir des signaux des détecteurs (3), dans lequel les signaux reçus des détecteurs (3) indiquent le degré auquel la lumière introduite dans le crâne du sujet est absorbée par les couches extracérébrales et cérébrales, ledit contrôleur (11) étant configuré pour soustraire le signal d'absorbance provenant du détecteur proximal (D1) du signal d'absorbance provenant du détecteur distal (D2) afin de fournir un signal d'absorbance pour les couches cérébrales du cerveau du patient, ledit signal d'absorbance des couches cérébrales étant représentatif - lorsque ladite sonde est fixée à la tête d'un sujet - de la lumière provenant des émetteurs qui a été modulée par la pulsation des artères cérébrales du sujet, les changements de pression entourant les artères cérébrales affectant la morphologie et d'autres caractéristiques dudit signal d'absorbance des couches cérébrales ;
le système comprenant en outre un module de modélisation (35) configuré pour établir un modèle mettant en relation des caractéristiques d'un signal optique avec la pression intracrânienne, le signal optique étant représentatif du degré auquel la lumière introduite dans le crâne d'un sujet est absorbée par le cerveau (8) du sujet, dans lequel ledit modèle comprend une fonction de prédiction entraînée sur des données d'essais cliniques étiquetées par des valeurs cibles de pression intracrânienne (PIC) obtenues au moyen d'une méthode de référence, les poids d'entrée du modèle étant ajustés au cours de l'entraînement afin de réduire l'erreur entre les valeurs de PIC prédites et les valeurs de PIC cibles ;
un module d'extraction de caractéristiques (37) permettant d'extraire une ou plusieurs caractéristiques du signal d'absorbance des couches cérébrales dérivé des signaux optiques délivrés par lesdits détecteurs (3), et
un module de prédiction de la pression intracrânienne (39) permettant d'introduire lesdites caractéristiques du signal dans ledit modèle et de délivrer une indication de la pression intracrânienne conformément audit modèle.

2. Système selon l'une quelconque des revendications précédentes, dans lequel lesdits émetteurs (2) sont configurés pour pouvoir émettre de la lumière infrarouge/infrarouge.

3. Système selon la revendication 2, dans lequel lesdits émetteurs optiques (2) sont configurés pour émettre de la lumière à une pluralité de longueurs d'onde différentes.

4. Système selon l'une quelconque des revendications précédentes, dans lequel lesdits émetteurs optiques (2) sont configurés pour émettre de la lumière à une longueur d'onde choisie parmi une ou plusieurs des longueurs d'onde suivantes : 810 nm, 770 nm, 855 nm, 880 nm, 780 nm, 805 nm, 850 nm et 870 nm.

5. Système selon la revendication 4, dans lequel la sonde (1) comprend quatre émetteurs (2), un premier desdits émetteurs (S1) étant configuré pour émettre de la lumière ayant une longueur d'onde d'environ 810 nm, un deuxième desdits émetteurs (S2) étant configuré pour émettre de la lumière ayant une longueur d'onde d'environ 770 nm, un troisième desdits émetteurs (S3) étant configuré pour émettre de la lumière ayant une longueur d'onde d'environ 855 nm, et un quatrième desdits émetteurs (S4) étant configuré pour émettre de la lumière ayant une longueur d'onde d'environ 880 nm.

6. Système selon l'une quelconque des revendications précédentes, dans lequel ledit modèle est établi au moyen d'une technique de régression à plusieurs variables.

7. Système selon l'une quelconque des revendications précédentes, dans lequel ledit module d'extraction de caractéristiques (37) est configuré pour extraire l'une quelconque ou plusieurs des caractéristiques suivantes dudit signal d'absorbance : amplitude du pouls, pente de montée, temps de montée, taux de décroissance diastolique précoce, largeur du pouls, aire sous la courbe en phase diastolique tardive et lumière rétrodiffusée totale.

8. Système selon l'une quelconque des revendications précédentes, comprenant en outre un module de traitement/filtrage des signaux (41) configuré pour traiter et/ou filtrer les signaux générés par lesdits détecteurs (3).
